(19) Europäisches Patentamt / European Patent Office / Office européen des brevets

(11) **EP 3 739 035 A1**

(12) **EUROPEAN PATENT APPLICATION**
published in accordance with Art. 153(4) EPC

(43) Date of publication:
**18.11.2020 Bulletin 2020/47**

(21) Application number: **18906806.7**

(22) Date of filing: **17.12.2018**

(51) Int Cl.:
*C12M 1/26* *(2006.01)*      *G01N 35/10* *(2006.01)*

(86) International application number:
**PCT/JP2018/046339**

(87) International publication number:
**WO 2019/163270 (29.08.2019 Gazette 2019/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.02.2018 JP 2018027699**

(71) Applicant: **Yamaha Hatsudoki Kabushiki Kaisha Iwata-shi, Shizuoka-ken 438-8501 (JP)**

(72) Inventors:
• **SAKAMOTO, Masaru**
  **Iwata-shi, Shizuoka-ken 438-8501 (JP)**
• **KUMAGAI, Takahiko**
  **Iwata-shi, Shizuoka-ken 438-8501 (JP)**

(74) Representative: **Müller-Boré & Partner Patentanwälte PartG mbB Friedenheimer Brücke 21 80639 München (DE)**

(54) **DEVICE FOR PICKING UP BIOLOGICAL SUBJECT**

(57)      A cell transfer device (S) includes: a head (61) with a tip (10) attached to a lower end; a control unit (7) that performs a suction operation of causing the tip (10) to suck a cell (C) by controlling an operation of the head (61); and an input unit (75) that can input information to the control unit (7). The input unit (75) receives, as suction control information (D1), at least one of first information regarding a mode of containers (2, 3), second information regarding a culture medium, and third information regarding the cell. When performing the suction operation, the control unit (7) controls a movement amount of the tip (10) in an up-and-down direction, and/or a suction amount of sucking the cell (C) and the culture medium (L) from a suction hole (t) into the tip (10) based on the suction control information (D1).

FIG.10

## Description

### Technical Field

The present invention relates to a pickup device that picks up, for example, a biological subject such as cells or cell clusters accommodated in a container from the container by using a suction tip.

### Background Art

For example, in medical and biological research applications, work of selecting biological subjects such as cells or cell clusters in a transfer source container and transferring the selected biological subjects to a destination container may be performed. As the transfer source container, for example, a plate having a large number of wells intended to individually accommodate biological subjects, a petri dish having a flat bottom surface on which a large number of biological subjects land, and the like are illustrated.

In the work of transfer, work of capturing an image of the biological subjects accommodated in the transfer destination container with an image capturing device, selecting a desired biological subject cell based on the obtained image, and sucking (picking up) the selected biological subject with a tip and transferring to a transfer destination container is performed (for example, Patent Literature 1). The tip has a suction hole for a biological subject at a lower end, and is attached to a head movable in the horizontal direction and the up-and-down direction. The head includes a suction mechanism that generates suction force in the suction hole.

When causing the tip to suck the biological subject, a suction sequence is performed in which the head is lowered to bring the suction hole of the tip closer to the target biological subject, and the suction mechanism generates the suction force in the suction hole. This suction sequence causes the biological subject together with the culture medium to be sucked into the tip. Conventionally, the suction sequence is performed under a constant condition regardless of the type of container or culture medium, the nature of the biological subject, and the like. That is, under any condition, the suction hole of the tip is brought closer to the biological subject by a constant distance, and the biological subject is sucked with a constant suction amount. With such a constant suction sequence, a suction failure may occur, in which suction of the biological subject fails, or a plurality of biological subjects are sucked simultaneously.

### Citation List

### Patent Literature

Patent Literature 1: WO 2015/087371 A

## Summary of Invention

### Problems to be Solved by the Invention

An object of the present invention is to provide a device for picking up a biological subject that can prevent suction failures when causing the tip to suck the biological subject accommodated in the container.

A device for picking up a biological subject according to one aspect of the present invention includes: a base on which a container having an upper surface opening is mounted, the container accommodating the biological subject and a culture medium; a head placed above the base and movable in a horizontal direction and an up-and-down direction, and having a tip including a suction hole that sucks the biological subject from the container and being attached to a lower end of the head, the head including a suction mechanism that generates suction force in the suction hole; a control unit configured to cause the tip to perform a suction operation of sucking the biological subject by controlling an operation of the head; and an input unit configured to input information to the control unit, wherein the input unit receives at least one of first information regarding a mode of the container, second information regarding the culture medium, and third information regarding the biological subject as suction control information, and when performing the suction operation, based on the suction control information, the control unit controls a movement amount of the tip attached to the head in the up-and-down direction, and/or a suction amount of sucking the biological subject and the culture medium from the suction hole into the tip.

### Brief Description of Drawings

FIG. 1 is a diagram schematically showing a configuration example of a cell transfer device to which a device for picking up a biological subject according to the present invention is applied.
FIG. 2A is a cross-sectional view of a tip attached to a head, and a diagram showing a movement mechanism and a suction mechanism for the tip, FIG. 2B is a cross-sectional view of the tip during a suction operation, and FIG. 2C is an enlarged view of a q portion of FIG. 2B.
FIG. 3A is an explanatory diagram of a suction range of the tip, and FIG. 3B is a map showing a relationship between a suction success rate of the biological subject by the tip, and a height and a distance.
FIG. 4 is a diagram schematically showing a first example of a suction sequence according to the present embodiment.
FIGS. 5A and 5B are diagrams showing operations of the first example.
FIG. 6 is a diagram schematically showing a second example of the suction sequence.

FIGS. 7A and 7B are diagrams showing operations of the second example.

FIGS. 8A and 8B are diagrams schematically showing a third example of the suction sequence.

FIG. 9 is a diagram schematically showing a fourth example of the suction sequence.

FIG. 10 is a block diagram showing a configuration of the cell transfer device.

FIG. 11 is a view showing a display example on a display unit.

FIG. 12 is a flowchart of a cell pickup operation using the cell transfer device.

**Description of Embodiment**

An embodiment of the present invention will be described in detail below with reference to the drawings. A device for picking up a biological subject according to the present invention can pick up (suck) a variety of biological subjects. As the biological subject to which the present invention can be applied, a cell of biological origin can be typically illustrated. Examples of the cell of biological origin here include a single cell (cell) such as a blood cell and single cell, tissue fragment such as Histoculture and CTOS, cell aggregation cluster such as spheroids and organoids, individuals such as zebrafish, nematodes, fertilized eggs, and 2D or 3D colony. In addition, tissues, microorganisms, small species, and the like can be illustrated as the biological subject. The embodiment described below shows an example in which the biological subject is cells or a cell aggregation cluster formed by aggregating several to several hundred thousand cells (hereinafter, collectively referred to simply as "cell C").

[Overall structure of cell transfer device]

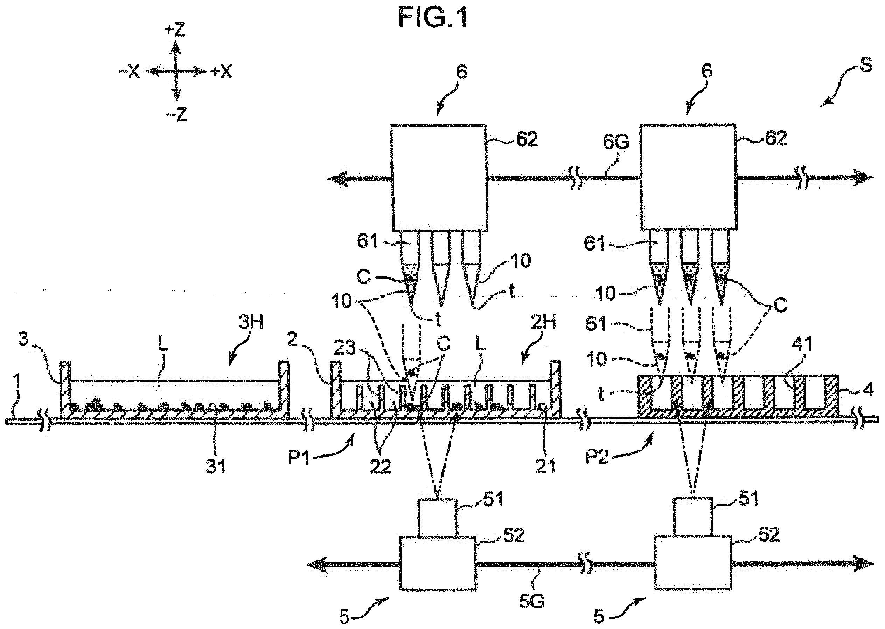

FIG. 1 is a diagram schematically showing an overall configuration example of a cell transfer device S to which a device for picking up a biological subject according to the present invention is applied. Here, the cell transfer device S that transfers a cell C between two containers, specifically between a first container 2 or a second container 3, and a microplate 4 is illustrated.

The cell transfer device S includes a translucent base 1 having an upper surface, which is a horizontal mounting surface, a camera unit 5 placed below the base 1, and a head unit 6 placed above the base 1. Either of the first container 2 having lattice-shaped wells or the petri dish-type second container 3 is placed at a first mounting position P1 of the base 1. The microplate 4 is mounted at a second mounting position P2. The first container 2 and the second container 3 are one example of the "container" of the present invention. Note that FIG. 1 shows only two containers 2 and 3, but in the present embodiment, containers of other formats are also applicable. That is, it is possible to mount any one or a plurality of containers selected from the group of three or more containers at the first mounting position P1 of the base 1.

The head unit 6 includes a plurality of heads 61 to which tips 10 that each suck and discharge the cell C are attached, the heads 61 being movable in a Z direction (up-and-down direction). The camera unit 5 and the head unit 6 are movable in the X direction (horizontal direction) and the direction perpendicular to the plane of FIG. 1 (Y direction). The first container 2 or the second container 3 and the microplate 4 are mounted on an upper surface of the base 1 within a movable range of the head unit 6.

Roughly, the cell transfer device S is a device in which each of the plurality of tips 10 picks up the cell C individually from the first container 2 or the second container 3 holding a large number of cells C, and transfers the picked cell C to the microplate 4, and the plurality of tips 10 individually or simultaneously discharge the cells C to wells 41 of the microplate 4. Before the suction of the cells C, the cells C held in the first container 2 or the second container 3 are captured by the camera unit 5, and a selection operation of selecting good quality cells C to be transferred to the microplate 4 is performed.

Each part of the cell transfer device S will be described below. The base 1 is a rectangular flat plate having predetermined rigidity, and part or all of which is formed of a translucent material. The preferred base 1 is a glass plate. The base 1 is formed of a translucent material such as a glass plate, thereby allowing the camera unit 5 placed below the base 1 to capture the first and second containers 2 and 3 and the microplate 4 placed on an upper surface of the base 1 through the base 1.

The first container 2 and the second container 3 are containers serving as a transfer source of the cells C. The first container 2 and the second container 3 are containers having upper surface openings 2H and 3H, and accommodating cells C and a culture medium L therefor. The culture medium 3 and the cells C are injected into the first container 2 and the second container 3 through the upper surface openings 2H and 3H, and the cells C are sucked by the suction tips 10. The first container 2 and the second container 3 made of a translucent resin material or glass is used. This is to allow the camera unit 5 to observe the cells C supported in the first container 2 and the second container 3.

The first container 2 includes a plurality of wells 22 including lattice-shaped compartments for accommodating the cells C inside the container. Lattice walls 23 that partition the wells 22 are placed on a bottom surface 21 of the first container 2. The wells 22 are compartments intended to hold the cells C individually when a cell suspension in which a large number of cells C are dispersed in the culture medium L is injected into the first container 2 from the upper surface opening 2H. The cell C lands on the bottom surface 21 in each well 22.

Meanwhile, the second container 3 is a petri dish type container that does not include lattice-shaped compartments. The entire bottom surface 31 of the second container 3 includes a flat plane portion. Note that rough compartments each intended to accommodate a plurality of cells C, for example, a wall portion that divides into two or four compartments may be provided on the bottom surface 31. When the cell suspension is injected into the second container 3 from the upper surface opening 3H, a plurality of cells C land on the bottom surface 31 (plane portion).

The microplate 4 includes a plurality of wells 41 into which the cells C are discharged. The wells 41 are each a bottomed hole opened on an upper surface of the microplate 4. One well 41 accommodates a required number of cells C together with the liquid culture medium 3. Usually, the required number is one. The microplate 4 made of a translucent resin material or glass is also used. This is to allow the camera unit 5 placed below the microplate 4 to observe the cells C supported in the well 41.

The camera unit 5 captures an image of the cells C held in the first or second container 2 or 3 or the microplate 4 from the lower surface side thereof, and includes a lens unit 51 and a camera body 52. The lens unit 51 is an object lens used in an optical microscope, and includes a lens group that forms a light image with a predetermined magnification and a lens barrel that accommodates the lens group. The camera body 52 includes an image capturing element such as a CCD image sensor. The lens unit 51 forms a light image of an image capturing target on a light receiving surface of the image capturing element. The camera unit 5 is movable in the X and Y directions below the base 1 along a guide rail 5G extending in the left-right direction parallel to the base 1. In addition, the lens unit 51 is movable in the Z direction for a focusing operation.

The head unit 6 is provided for picking up the cells C from the first and second containers 2 and 3 and transferring the cells C to the microplate 4, and includes the plurality of heads 61 and a head body 62 to which the heads 61 are assembled. At the tip of each head 61, the suction tip 10 that sucks (picks up) and discharges one or more cells C is attached. The head body 62 holds the heads 61 so as to be raised and lowered in the +Z and -Z directions (up-and-down direction), and is movable in the +X and -X directions (horizontal direction) along a guide rail 6G. Note that the head body 62 is also movable in the Y direction. That is, the head 61 is movable in the XYZ directions.

[Details of tip and head]

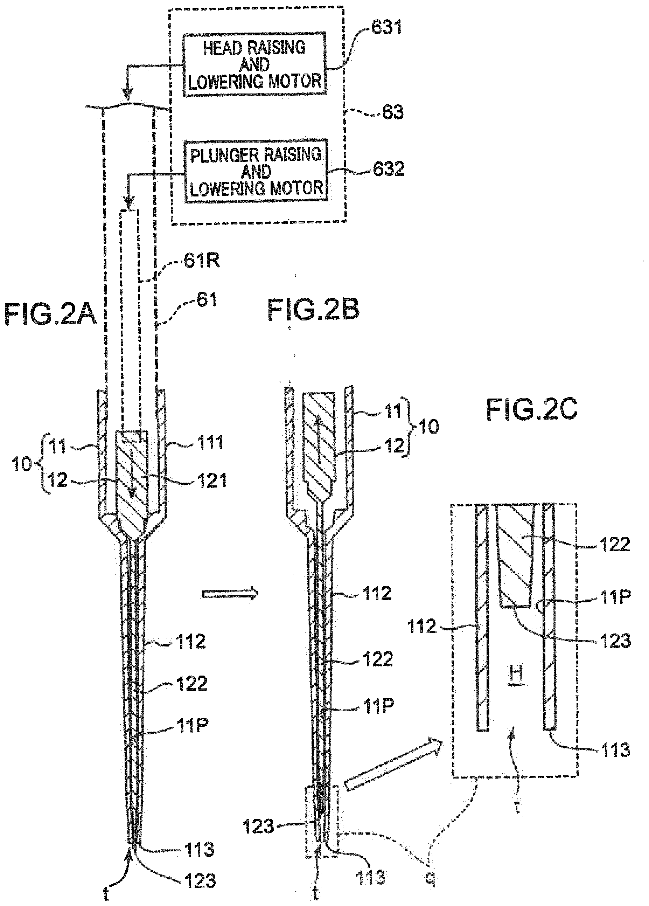

FIG. 2A is a cross-sectional view of the tip 10 attached to the head 61, and a diagram showing a movement mechanism and a suction mechanism for the tip 10. The tip 10 includes an assembly of a syringe 11 and a plunger 12. The syringe 11 internally includes a tubular passage 11P serving as a suction path for the cells C. The plunger 12 moves forward and backward in the tubular passage 11P while sliding in contact with an inner peripheral wall of the syringe 11 that defines the tubular passage 11P.

The syringe 11 includes a syringe proximal end 111 including a cylindrical body having a large diameter, and a syringe body portion 112 including a long cylindrical body having a small diameter. The tubular passage 11P is formed in the syringe body portion 112. A syringe distal end 113, which is a lower end of the syringe body portion 112, is provided with a suction hole t serving as an opening through which the cells C accommodated in the containers 2 and 3 are suctioned or discharged. One end of the tubular passage 11P is connected to the suction hole t. The syringe proximal end 111 is connected to the other end side of the syringe body portion 112 via a tapered portion. An upper end portion of the syringe proximal end 111 is fitted and attached to a lower end of the head 61.

The plunger 12 includes a plunger proximal end 121 including a cylindrical body, a needle-shaped plunger body portion 122 connected below the plunger proximal end 121, and a plunger distal end 123 that is a lower end of the plunger body portion 122. An outer diameter of the plunger proximal end 121 is set to be smaller than an inner diameter of the syringe proximal end 111 by a predetermined length. An outer diameter of the plunger body portion 122 is set to be slightly smaller than an inner diameter of the tubular passage 11P.

The plunger 12 is assembled with respect to the syringe 11 in such a manner that the plunger proximal end 121 is accommodated in the syringe proximal end 111 and the plunger body portion 122 is inserted into the tubular passage 11P of the syringe body portion 112. In the state of FIG. 2A in which the plunger body portion 122 is inserted most deeply into the syringe body portion 112, the plunger distal end 123 protrudes from the suction hole t. A rod 61R, which is movable in the up-and-down direction in an inner space of the head 61, is attached to the upper end of the plunger proximal end 121.

Head motors 63 that function as a movement mechanism of the tip 10 in the up-and-down direction and a suction mechanism that generates suction force in the suction hole t of the tip 10 are attached to the head 61. The head motors 63 are a plurality of motors incorporated in the head body 62, and include a head raising and lowering motor 631 and a plunger raising and lowering motor 632. The plunger raising and lowering motor 632 is one example of the suction mechanism.

The head raising and lowering motor 631 is a motor serving as a drive source that raises and lowers the head 61 with respect to the head body 62. When the head 61 is raised and lowered by the drive of the head raising and lowering motor 631, the tip 10 attached to the lower end of the head 61 is also raised and lowered. That is, the height position of the suction hole t of the tip 10 can be set at a desired position by controlling the operation of the head raising and lowering motor 631.

The plunger raising and lowering motor 632 is a motor serving as a drive source that raises and lowers the rod 61R in the internal space of the head 61. When the rod 61R is raised and lowered by the drive of the plunger raising and lowering motor 632, the plunger 12 attached to this rod 61R is also raised and lowered. When the plunger 12 is raised with respect to the syringe 11, suction force is generated in the suction hole t. When the plunger 12 is lowered, discharge force is generated in the suction hole t. That is, by controlling the operation of the plunger raising and lowering motor 632, the suction operation and discharge operation of the cell C by the tip 10 can be controlled.

FIG. 2A shows a state in which the plunger 12 is most lowered. This state is a state before the suction of the cells C is performed or a state after the cells C sucked in the tip 10 are discharged. The plunger distal end 123 protrudes slightly below the syringe distal end 113. FIG. 2B shows a state in which the plunger 12 is raised by a predetermined height. This state is a state of the tip 10 during the suction operation for suctioning the cells C. FIG. 2C shows an enlarged view of a q portion (peripheral portion of the suction hole t) of FIG. 2B.

During the suction operation, the plunger distal end 123 is retracted into the tubular passage 11P. At this time, suction force is generated in the suction hole t, and a fluid around the suction hole t, in the present embodiment, the culture medium L containing the cells C is sucked into suction space H formed in the tubular passage 11P because the plunger distal end 123 is retracted in the tubular passage 11P. That is, the culture medium L containing the cells C is held in the suction space H. After the suction operation, when the plunger 12 is moved downward, the fluid held in the suction space H is discharged from the suction hole t. A suction amount of the fluid can be adjusted by the raising height of the plunger 12. A suction speed of the fluid can be adjusted by the raising speed of the plunger 12. That is, the suction amount and the suction speed can be set at desired values by controlling the operation of the plunger raising and lowering motor 632.

[Suction characteristic of tip]

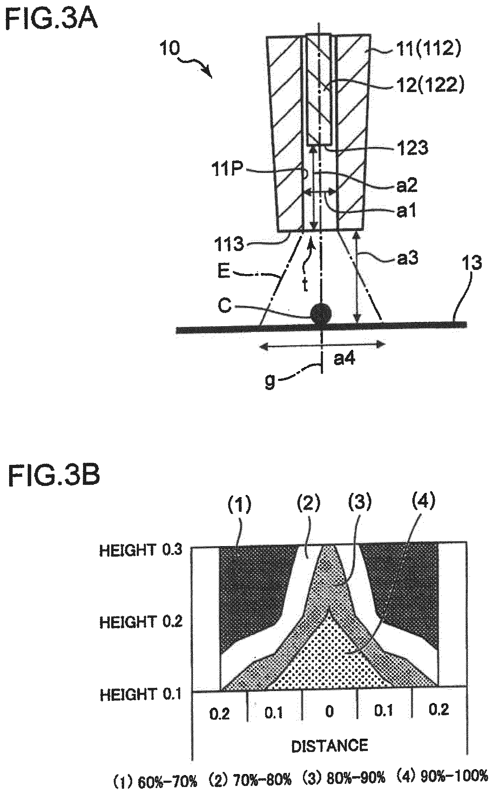

FIG. 3A s an explanatory diagram of a suction range of the tip 10, and FIG. 3B is a map showing a relationship between a suction success rate of the cell C by the tip 10, and a height and a distance. FIG. 3A shows a state in which the plunger 12 is raised relatively with respect to the syringe 11 and the plunger distal end 123 is retracted into the tubular passage 11P, that is, a state in which the suction operation is being performed. The suction hole t directly faces the cell C that has landed on a landing surface 13.

The suction amount of the liquid into the syringe 11, here, the suction amount of the culture medium containing the cells C is determined by an inner diameter a1 of the tubular passage 11P and a retraction length a2 of the plunger distal end 123. In the liquid around the syringe distal end 113, a suction range E, which is a range in which the liquid flows in a direction in which the liquid is taken into the suction hole t due to the generation of suction force to the suction hole t, is determined by a height a3 of the syringe distal end 113 with respect to the landing surface 13 and the suction amount. By one suction operation, the liquid of the volume roughly within the suction range E or the liquid of the volume larger than within the suction range E is sucked into the syringe 11. Therefore, if the suction operation is performed in a state where one or more cells C to be sucked are accommodated in the suction range E, the probability of sucking the cells C into the syringe 11 can be increased. That is, the cells C existing within a distance a4 (spot diameter of the suction range E) where the suction range E intersects the landing surface 13 can be sucked into the syringe 11.

FIG. 3B shows the suction success rate of the cells C when the viscosity of the culture medium and the suction amount are constant. The inner diameter a1 of the tubular passage 11P of the used syringe 11 is 0.18 mm. The horizontal axis corresponds to the distance a4 shown in FIG. 3A and indicates a distance (mm) from an axis g of the tubular passage 11P. The vertical axis indicates a height (mm) corresponding to the height a3. For example, the suction success rate of the height 0.1 to 0.3 at the point of distance = 0 indicates the suction success rate in the height direction on the axis g shown in FIG. 3A.

A region (1) of FIG. 3B is a region where the height a3 is high and the cell C is spaced from the axis g, and the suction success rate of the cell C is the lowest (60% to 70%). In a region (2) inside the region (1), the suction success rate is slightly increased (70% to 80%). In a region (3) where the height is 0.3 mm or less and the distance is 0.2 mm or less, the suction success rate increases to 80% to 90%. Furthermore, in a region (4) where the height is 0.2 mm or less and the distance is 0.1 mm or less, the suction success rate reaches 90% to 100%. If such a suction characteristic is experimentally obtained for various tips 10, by setting the suction amount and the height a3, the suction range E (distance a4) where the cell C can be sucked can be set at a desired range.

When causing the tip 10 to suck the cell C, a suction sequence is performed in which the suction hole t of the tip 10 is brought close to the target cell C to generate suction force in the suction hole t. This suction sequence causes the cell C to be sucked into the tip 10 together with the culture medium. At this time, the approach distance of the suction hole t to the target cell C is set with reference to the suction range E. Conventionally, the suction sequence is performed under a constant condition regardless of the type of container or culture medium accommodating the cell C, the property of the cell C, and the like. That is, under any condition, the suction hole t of the tip 10 is brought close to the cell C by a constant distance and sucked with a constant suction amount. In such a constant suction sequence, a suction failure may occur in which suction of the cell C fails or a plurality of cells C are suctioned at the same time. The present embodiment prevents the suction failure by appropriately setting the suction sequence according to the type of container or culture medium, the property of the cell C, and the like. Several specific examples of the suction sequence will be described below.

[First example of suction sequence]

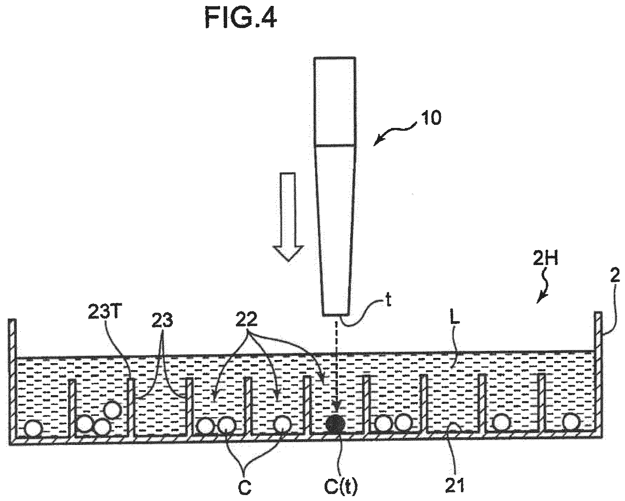

FIG. 4 is a diagram schematically showing a first example of the suction sequence according to the present embodiment. Here, a case is illustrated where the container serving as the transfer source of the cell C is the first container 2 in a mode of the container including a plurality of wells 22. The lattice walls 23 that partition the plurality of wells 22 are erected on the bottom surface 21 inside the first container 2. The culture medium L is injected into the first container 2. An injection amount of the culture medium L is an amount that causes the upper surface of the culture medium L to be positioned higher than an upper end opening edge 23T of the wells 22. That is, this is a state where the lattice walls 23 are immersed in the culture medium L.

The cells C are injected from the upper surface opening 2H of the first container 2 in a state of the cell suspension in which the cells C are dispersed in the culture medium L. Each well 22 is intended to accommodate one cell C. However, since a form of injecting the cell suspension is adopted, actually, one cell C or two or more cells C may enter one well 22, or some well 22 may fail to accommodate any cell C. Some wells 22 may accommodate only one cell C. Since it is easy to determine the quality of the isolated cell C by image capturing with the camera unit 5, the cell determined to be "good" among these cells is subjected to suction (transfer) by the tip 10. FIG. 4 shows the tip 10 having XY coordinates aligned with one well 22 accommodating one cell C (t) serving as a suction target for performing the suction operation, the tip 10 being placed above the well 22.

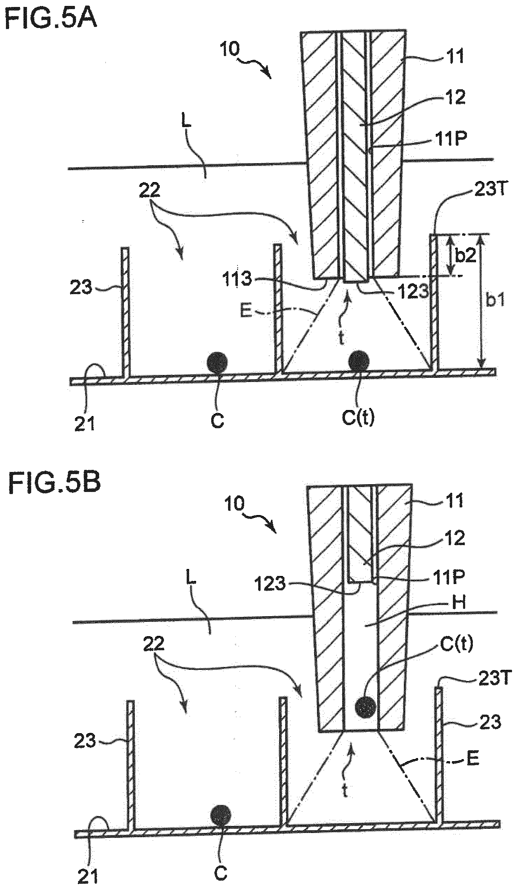

FIGS. 5A and 5B are diagrams showing operations of the first example of the suction sequence. When performing the suction operation with the tip 10 on the target cell C (t) accommodated in the well 22 immersed in the culture medium L, the tip 10 is lowered such that the suction hole t enters the well 22. In detail, as shown in FIG. 5A, a downward movement amount of the tip 10 is controlled such that the position of the suction hole t flush with the syringe distal end 113 becomes a position above the target cell C (t) accommodated in the well 22 and below the upper end opening edge 23T of the well 22. That is, if the height of the upper end opening edge 23T from the bottom surface 21 is b1, the suction hole t is lowered to a position closer to the bottom surface 21 by a predetermined approach length b2 than the height b1.

The approach length b2 is selected such that the lower end of the suction range E of the tip 10 reaches the bottom surface 21 with which the target cell C (t) is in contact. The suction hole t is preferably positioned directly above the target cell C (t). Note that if the approach length b2 is too deep, because of an error of axis control precision of the head 61 to which the tip 10 is attached or the like, the syringe distal end 113 or the plunger distal end 123 may touch and damage the target cell C (t). Therefore, the approach length b2 is set at such a depth that the tip 10 does not come into contact with the target cell C (t) even in consideration of the precision error.

FIG. 5A shows the tip 10 before starting the suction operation. That is, the plunger 12 is most lowered, and the plunger distal end 123 slightly protrudes from the suction hole t of the syringe distal end 113. Meanwhile, FIG. 5B shows the tip 10 having completed the suction operation. The plunger distal end 123 is retracted into the tubular passage 11P by a predetermined retraction length. By this retraction operation, suction force is generated in the suction hole t, and the target cell C (t) is sucked together with the culture medium L into the suction space H generated near the lower end of the tubular passage 11P due to the retraction.

In the first example of the suction sequence, the suction operation is performed with the suction hole t having entered the well 22 accommodating the target cell C (t). This makes it possible to make a flow of the culture medium L accompanying the suction operation unlikely to occur in the peripheral well 22 adjacent to the well 22. Therefore, when the suction operation is performed on the target cell C (t), it is possible to prevent the cell C accommodated in the peripheral well 22 from being sucked incidentally. That is, only the target cell C (t) can be sucked into the tip 10.

Note that by the suction operation, the culture medium L corresponding to the suction range E is introduced into the tip 10 in the well 22 in which the target cell C (t) has existed. In order to fill the introduced culture medium L, a flow is caused by the culture medium L entering the well 22 through a gap between the tip 10 and the upper end opening edge 23T. If this flow is large, the tip 10 may simultaneously suck the cell C accommodated in the adjacent well 22. Therefore, the retraction length of the plunger distal end 123, that is, the suction amount of the culture medium L from the suction hole t is preferably smaller than the volume of one well 22. With this configuration, it is possible to control the occurrence of flow that draws in the cell C from the adjacent well 22.

[Second example of suction sequence]

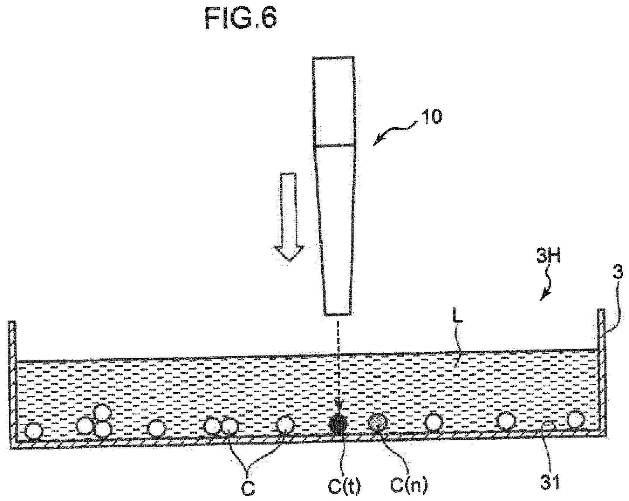

FIG. 6 is a diagram schematically showing a second example of the suction sequence according to the present embodiment. Here, a case is illustrated where the container serving as the transfer source of the cell C is the second container 3 of an example of petri dish type that does not include lattice-shaped compartments. As described above, the second container 3 has the bottom surface 31 including a flat plane portion. Since the cell suspension in which the cells C are dispersed in the culture medium L is introduced from the upper surface opening 3H, the plurality of cells C land on the bottom surface 31. The cells C dispersively land on the bottom surface 31 in an isolated state or in a state where the plurality of cells C are aggregated. FIG. 6 shows a state in which the tip 10 with the XY coordinates aligned with one cell C (t) serving as the suction target for performing the suction operation is placed above the target cell C (t). Note that the other cell C existing at the position closest to the target cell C (t) is called an adjacent cell C (n).

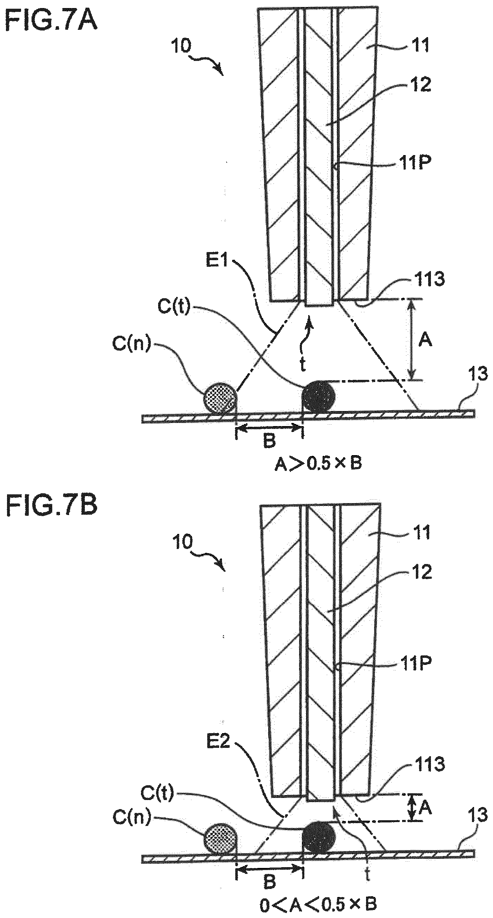

FIGS. 7A and 7B are diagrams showing operations of the second example of the suction sequence. In the second container 3 that does not include the lattice walls 23 as in the first container 2 of the first example, because of the suction flow generated when the target cell C (t) is sucked, the adjacent cell C (n) is easily sucked simultaneously. In order to regulate the suction range E so as to prevent such simultaneous suction, the height position of the suction hole t with respect to the target cell C (t) is set. Specifically, when the distance between the suction hole t and the target cell C (t) is A and the distance between the target cell C (t) and the adjacent cell C (n) is B, the downward movement amount of the tip 10 is controlled such that the suction hole t is positioned at a height position that satisfies the relationship:

$$0 < \text{distance A} < 0.5 \times \text{distance B.}$$

FIG. 7A shows a state immediately before the suction operation is performed with the suction hole t placed at the height position (A > 0.5 × B) that does not satisfy the above conditional expression. In this case, the tip 10 has a suction range E1 according to the height position thereof. Generally, at a height position where the distance A is 1/2 or more of the distance B, the probability that the adjacent cell C (n) is included in the suction range E1 is high. Therefore, if the suction operation is performed, the probability that not only the target cell C (t) but also the adjacent cell C (n) will be simultaneously suctioned is high, which is not preferable.

In contrast, FIG. 7B shows a state immediately before the suction operation is performed with the suction hole t placed at the height position (0 < A < 0.5 × B) that satisfies the above conditional expression. That is, the suction hole t is brought close to the target cell C (t) such that the distance A is 1/2 or less of the distance B. In this case, the tip 10 has a suction range E2 narrower than the suction range E1 of FIG. 7A. With the suction range E2 regulated in this way, the adjacent cell C (n) can be set outside the suction range E2, and the simultaneous suction of the adjacent cell C (n) can be prevented. As in the second example, when performing the suction operation on the second container 3 in which the plurality of cells C exist on the planar bottom surface 31, by setting the suction sequence of setting the height position of the suction hole t by considering the distance B between the target cell C (t) and the adjacent cell C (n), it is possible to increase the probability of causing only the target cell C (t) to be sucked into the tip 10.

[Third example of suction sequence]

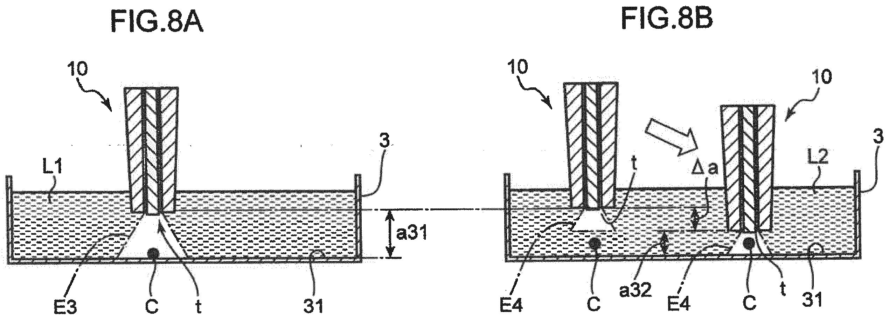

FIGS. 8A and 8B are diagrams schematically showing a third example of the suction sequence. The third example shows an example in which the suction sequence is changed depending on a difference in viscosity of the culture medium L. Generally, as the viscosity of the culture medium L increases, the suction range E of the tip 10, that is, the range where the suction flow is generated in the surrounding culture medium L by generating suction force in the suction hole t becomes narrower. There are also circumstances that as the viscosity of the culture medium L increases, unless the amount of the culture medium L sucked into the tip 10 is reduced, it becomes difficult to discharge the cell C once sucked into the tip 10 from the suction hole t. In view of these points, the third example shows an example in which, when there are a plurality of culture media with different viscosity as the culture medium to be accommodated in the container, the suction sequence is set such that the suction hole t is brought closer to the cell C as the viscosity of the culture medium increases.

It is assumed that the second container 3 shown in FIG. 8A accommodates the first culture medium L1 having predetermined first viscosity and the cell C. Meanwhile, the second container 3 shown in FIG. 8B accommodates the second culture medium L2 having second viscosity higher than the first viscosity and the cell C. For example, the first culture medium L1 is a liquid culture medium, and the second culture medium L2 is a semisolid culture medium such as Matrigel (trade name of Corning Incorporated). As shown in FIG. 8A, when the cell C in the first culture medium L1 is sucked, the height of the suction hole t with respect to the bottom surface 31 is set at a predetermined height a31. In this case, a suction range E3 according to the height a31 and the suction amount is formed.

Meanwhile, as shown in FIG. 8B, when the suction operation is performed in the second culture medium L2 having higher viscosity than the first culture medium L1, a suction range E4 is narrower than the suction range E3. Therefore, if the suction hole t is set at the same height a31 as in the case of the first culture medium L1 and the suction operation is performed, the cell C having landed on the bottom surface 31 cannot be sucked into the tip 10. Therefore, when the suction operation is performed on the cell C in the second culture medium L2, the height position of the suction hole t is set at a32 lower than the height a31 for the first culture medium L1 by Δa. That is, the suction hole t is set at a position closer to the cell C by Δa, and the suction operation is performed. With this operation, the cell C is included in the suction range E4, and the cell C can be sucked into the tip 10. In the third example described above, even if the use of culture media L1 and L2 having different viscosity is assumed, it is possible to prevent the occurrence of a suction failure of the cell C.

[Fourth example of suction sequence]

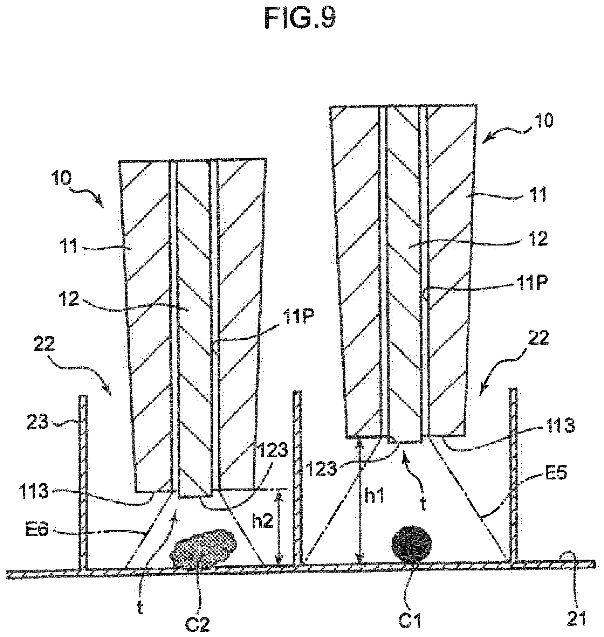

FIG. 9 is a diagram schematically showing a fourth example of the suction sequence. The fourth example shows an example in which the suction sequence is changed by the difference in adhesive strength of the cell C with respect to the bottom surfaces 21 and 31 of the first container 2 and the second container 3. Depending on the nature of the cell C, the adhesive strength with respect to the bottom surfaces 21 and 31 may differ. If the same suction sequence is applied to the cells C having different adhesive strength, the probability of occurrence of a suction failure of the cell C increases. The fourth example shows an example in which, when performing the suction operation on the cell C having stronger adhesive strength, the suction sequence is set such that the suction hole t is brought closer to the cell C or the suction amount is increased.

FIG. 9 shows an example in which a first cell C1 (first biological subject) that lands on the bottom surface 21 with first adhesive strength, and a second cell C2 (second biological subject) that lands on the bottom surface 21 with second adhesive strength stronger than the first adhesive strength exist in respective two wells 22 of the first container 2. Here, an example is shown in which the height position of the suction hole t during the suction operation is changed according to the adhesive strength of the cell C with respect to the bottom surface 21. When causing the tip 10 to suck the first cell C1, the height of the suction hole t with respect to the bottom surface 21 is set at a predetermined height h1. In this case, a suction range E5 according to the height h1 and the suction amount is formed.

Meanwhile, when causing the tip 10 to perform the suction operation on the second cell C2 having higher adhesive strength than the first cell C1, the position of the suction hole t is set at a height h2 closer to the second cell C2 than the height h1 when the suction operation is performed on the first cell C1. With this configuration, a suction range E6 is apparently narrower than the suction range E5. However, if the suction amount to the tip 10 is the same, that is, if the retraction amount of the plunger distal end 123 is the same, the suction force acting on the second cell C2 can be larger than the suction force on the first cell C1. Therefore, even when the second cell C2 lands on the bottom surface 21 more strongly than the first cell C1, the second cell C2 can be sucked into the tip 10. In this way, in the fourth example, the occurrence of a suction failure can be prevented even when the suction of the cell C having different adhesive strength with respect to the bottom surface 21 is assumed.

Note that in the fourth example, during the suction operation by the tip 10, as long as the suction force acting on the second cell C2 having higher adhesive strength can be larger than the suction force on the first cell C1, various suction sequences can be adopted. For example, a mode can be adopted in which the suction amount of the culture medium L into the tip 10 is larger when the second cell C2 is sucked than when the first cell C1 is sucked. In this case, the retraction amount of the plunger distal end 123 will be larger when the second cell C2 is sucked than when the first cell C1 is sucked.

Alternatively, the suction force may be increased by increasing the raising speed of the plunger 12 to increase the suction speed of the culture medium L from the suction hole t. When the suction operation by the tip 10 is performed in a plurality of times, the total suction force may be increased by increasing the number of times of suction. Furthermore, a method of increasing the suction force and the operation of bringing the suction hole t of the fourth example closer to the second cell C2 may be used together.

In the fourth example, attention has been paid to the adhesive strength of the cell C with respect to the bottom surface 21, but the suction sequence may be changed according to another factor of the cell C. For example, the suction sequence may be changed depending on the size and shape of the cell C, the posture of accommodation in the first container 2 and the second container 3, and the like. For example, the cell C having a nearly spherical shape can be sucked into the tip 10 with relatively weaker suction force than the cell C having a distorted shape. For the cell C having an ellipsoid shape, when the cell C lands on the bottom surface 21 in an almost upright state, it is possible to suck the cell C into the tip 10 with relatively weaker suction force than when the cell C lands on the bottom surface 21 sideways. Furthermore, the suction sequence may be changed depending on how easily the cell C collapses, the number of cells to suck, and the like.

[Electric configuration of cell transfer device]

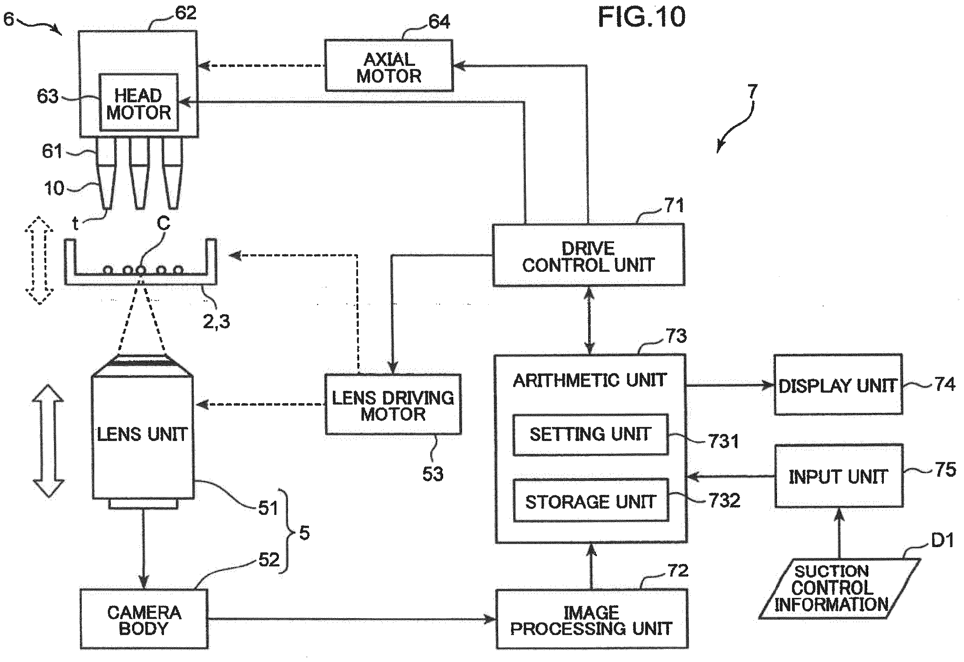

FIG. 10 is a block diagram showing an electric configuration of the cell transfer device S. The cell transfer device S includes the camera unit 5 that captures an image capturing target on an image capturing optical axis, a lens driving motor 53 that moves the lens unit 51 up and down, the head motors 63 in the head unit 6 that perform a picking up operation on the cell C in the first container 2 or the second container 3, an axial motor 64 that moves the head unit 6 in the XY direction, and a control unit 7. The control unit 7 controls the lens driving motor 53 to control the operation of the lens unit 51, performs predetermined processing on image information acquired by the camera body 52, and also controls the pickup operation and the transfer operation on the cell C by controlling the head motors 63 and the axial motor 64.

The lens driving motor 53 rotates forward or backward to move the lens unit 51 in an up-and-down direction with a predetermined resolution via a power transmission mechanism (not shown). By this movement, the focus position of the lens unit 51 is adjusted to the cells C having landed on the first container 2 or the second container 3. Note that as shown by the dotted line in FIG. 10, instead of the lens unit 51, the first container 2 or the second container 3 itself or the base 1, which is a stage on which the first container 2 and the second container 3 are mounted may be moved up and down by another motor that substitutes the lens driving motor 53.

The head motors 63 are motors that serve as a drive source for the raising and lowering operation of the heads 61 with respect to the head body 62, and for the operation of generating suction force and discharge force in the suction hole t of the tips 10 attached to the head 61. The axial motor 64 is a motor that serves as a drive source for moving the head unit 6 (head body 62) along the guide rail 6G (FIG. 1).

The control unit 7 includes, for example, a personal computer or the like, and operates to functionally include a drive control unit 71, an image processing unit 72, and an arithmetic unit 73 by executing a predetermined program.

The drive control unit 71 controls operations of the lens driving motor 53, the head motor 63, and the axial motor 64. Specifically, the drive control unit 71 gives control pulses for moving the lens unit 51 in an up-and-down direction at a predetermined pitch, for example, tens of $\mu$m pitch, to the lens driving motor 53 for the focusing operation. Also, although not shown in FIG. 10, the drive control unit 71 also controls an operation of a camera axis drive motor that moves the camera unit 5 along the guide rail 5G. Furthermore, the drive control unit 71 also controls mechanical operations of the head unit 6. The drive control unit 71 controls the head motors 63 to control the raising and lowering operation of the heads 61 and the operation of generating suction force or discharge force in the suction hole t of the tips 10.

The image processing unit 72 performs image processing such as edge detection processing and pattern recognition processing with feature amount extraction on image data acquired by the camera body 52. The image processing unit 72 acquires the image data of the first container 2 or the second container 3 supporting the cells C and recognizes the cells C existing on the first container 2 or the second container 3 by the image processing. Based on a result of the recognition, the cell C to be transferred is identified.

The arithmetic unit 73 mainly controls the operation of the head 61 to control the suction operation of causing the tip 10 to suck the cell C. The arithmetic unit 73 functionally includes a setting unit 731 that sets the suction sequence, and a storage unit 732 that stores various data and setting values. A display unit 74 is a display that displays an image of the cells C captured by the camera unit 5, displays the suction sequence, and the like. An input unit 75 is a terminal including a keyboard, a mouse, or the like, and receives various input information items for the control unit 7 from an operator.

The input unit 75 receives at least one of first information regarding a mode of the container accommodating the cells C, second information regarding the culture medium L, and third information regarding the cells C as suction control information D1. When causing the head 61 (tip 10) to perform the suction operation on the cell C, the setting unit 731 of the arithmetic unit 73 sets the movement amount of the tip 10 in the up-and-down direction and/or the suction amount for sucking the cell C and the culture medium L from the suction hole t into the tip 10 based on the suction control information D1 received by the input unit 75.

A specific example of the first information is information regarding the first container 2, which is a container of a mode in which there are a plurality of wells 22 shown in the first example of the suction sequence, and the second container 3, which is a container having the bottom surface 31 as the plane portion shown in the second example. The first information may be, for example, shape data itself of the container to use such as the size, volume, lattice width, and height, or may be data such as the type and model of the container. In the latter case, the shape data associated with the type or model number of the container planned to be used is stored in the storage unit 732 in advance. According to such first information, during the suction operation, the setting unit 731 sets the movement amount of the tip 10 in the up-and-down direction, that is, the height position of the suction hole t, and the suction amount of the culture medium L containing the cells C into the tip 10. In response to the setting, the drive control unit 71 controls the head motors 63 (head raising and lowering motor 631 and plunger raising and lowering motor 632) to cause the head 61 to perform the operation as set above.

For example, in the first example, as shown in FIGS. 5A and 5B, the setting unit 731 sets the downward movement amount of the tip 10 such that the suction hole t is positioned below the upper end opening edge 23T of the well 22. In the second example, as shown in FIGS. 7A and 7B, the setting unit 731 sets the downward movement amount of the tip 10 such that the suction hole t is positioned at a height position that satisfies the relationship of "0 < distance A < 0.5 × distance B."

A specific example of the second information is information regarding the amount of culture medium L as shown in the first example of the suction sequence, that is, the amount that is higher than the upper end opening edge 23T, and the viscosity of the culture medium L shown in the third example. The second information may be, for example, data itself such as whether the culture medium is liquid, semi-solid, or solid, type of material or the like, viscosity, amount, and the like, or data such as a management number of the culture medium. In the latter case, various data associated with the management number of the culture medium planned to be used is stored in the storage unit 732 in advance. The setting unit 731 sets the downward movement amount and the suction amount of the tip 10 according to such second information. For example, in the third example, as shown in FIGS. 8A and 8B, when performing the suction operation on the cell C in the second culture medium L2 having high viscosity, the setting unit 731 sets the downward movement amount of the tip 10 such that the suction hole t is positioned closer to the cell C as compared to the case of the first culture medium L1 having low viscosity.

A specific example of the third information is data such as the type, shape, size, color tone, and posture of accommodation of the cell C in the container, in addition to data regarding the cell C that is experimentally or empirically determined, like the adhesive strength of the cell C with respect to the bottom surface 21 as shown in the fourth example of the suction sequence. According to such third information, the setting unit 731 sets the downward movement amount and the suction amount of the tip 10. For example, in the fourth example, when sucking the second cell C having high adhesive strength, the setting unit 731 sets the downward movement amount of the tip 10 such that the suction hole t is closer as compared to the case of the first cell C1 having low adhesive strength. In addition to or instead of this, the setting unit 731 sets the suction amount of the culture medium L into the tip 10 to be larger when sucking the second cell C2 than when sucking the first cell C1.

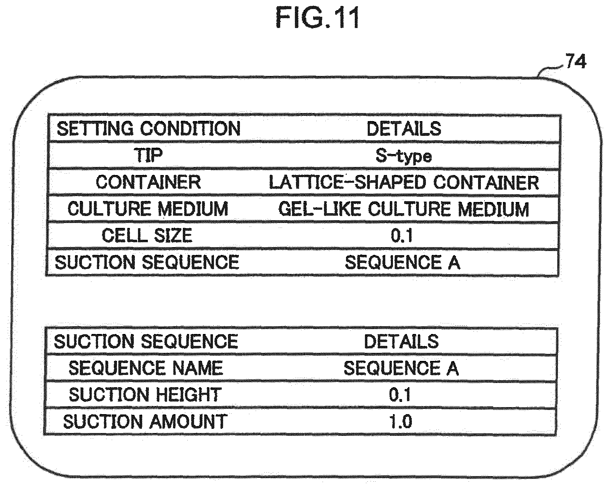

FIG. 11 is a view showing a display example on the display unit 74. Here, a setting example that is input from the input unit 75 to the setting unit 731 is shown regarding the tip 10, the containers 2 and 3, the culture medium L, and the cell C. The suction sequence selected according to these settings is "sequence A", and details of the "sequence A", that is, the suction height (height position of the suction hole t) and the suction amount are displayed. After checking this display, the operator gives the control unit 7 an instruction to perform the suction operation.

[Operation flow of cell transfer device]

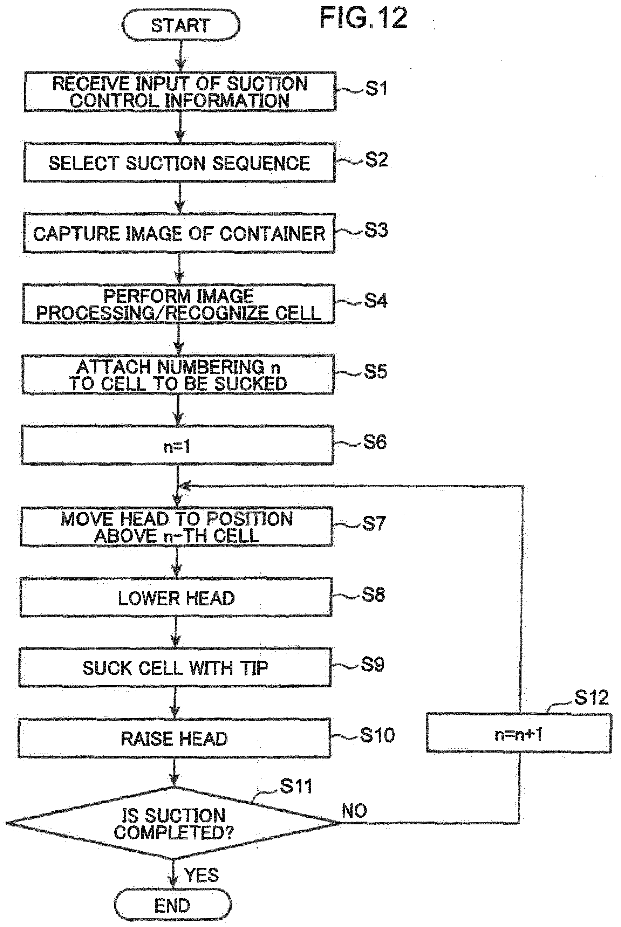

FIG. 12 is a flowchart of the cell suction operation using the cell transfer device S. With reference to FIG. 10 as well, when the processing starts, the input unit 75 receives the suction control information D1 including at least one of the first information to third information (step S1). According to the suction control information D1, the setting unit 731 of the arithmetic unit 73 sets the suction sequence. That is, the most suitable suction sequence in terms of the type and property of the container, the culture medium, the cell, and the like is selected (step S2).

Subsequently, the drive control unit 71 causes the camera unit 5 to capture an image of the cells C supported in the first container 2 or the second container 3, which is the container mounted at the first mounting position P1 (FIG. 1) (step S3). Specifically, the drive control unit 71 drives the lens unit 51 through the lens driving motor 53 to focus on the cells C in the container and performs the image capturing operation. Subsequently, the image processing unit 72 performs predetermined image processing on the image acquired by the camera body 52. By this image processing, the cells C appearing in the acquired image are recognized, and the arithmetic unit 73 derives the feature amount obtained by digitizing the external outline, size such as area and volume, shape, color tone, and the like of each cell C (step S4).

Furthermore, based on these feature amounts, the arithmetic unit 73 selects, from the cells C included in the image, the cell to be transferred to the microplate 4, that is, the cell C to be sucked by the tip 10 attached to the head 61. Numbering n is attached to the selected cell C (step S5). In response to the numbering, the drive control unit 71 sets n = 1 and starts the suction operation (step S6).

The drive control unit 71 operates the axial motor 64 to move the head 61 (tip 10) to the position above the n-th cell C in the XY directions (step S7). After the movement or during the movement in the XY directions, the drive control unit 71 starts the suction operation according to the suction sequence set in step S2. That is, the drive control unit 71 drives the head raising and lowering motor 631 (FIG. 2) of the head motors 63 to start lowering the head 61, and stops at the position where the suction hole t reaches the height position according to the setting value of the suction sequence (step S8). After the lowering of the head 61 is completed, or while the head 61 is lowered, the drive control unit 71 drives the plunger raising and lowering motor 632 (suction mechanism) to raise the plunger 12 such that the suction amount according to the setting value of the suction sequence is implemented. With this operation, the n-th cell C is sucked into the tip 10 (step S9). After or during the suction, the drive control unit 71 raises the head 61 (step S10).

Thereafter, the drive control unit 71 determines whether the suction of all the numbered cells C has been completed (step S11). When the suction is not yet completed (NO in step S11), the drive control unit 71 increments n by 1 (step S12), returns to step S7, and repeats the processing. On the other hand, when the suction is completed (YES in step S11), the processing ends.

As described above, with the cell transfer device S according to the present embodiment, the movement amount of the tip 10 in the up-and-down direction and/or the suction amount in the suction operation of the cell C is controlled according to the suction control information D1 received by the input unit 75. That is, the mode of the suction operation is changed according to the mode of the first container 2 and the second container 3, and the information about the culture medium L and the cell C (biological subject). Therefore, it is possible to set the optimum suction sequence according to the suction control information D1 instead of the uniform suction sequence of the cell C. As a result, it is possible to prevent the occurrence of a suction failure of the cell C.

[Invention included in the above embodiment]

Note that the above-described specific embodiment mainly includes the invention having the following configurations.

A device for picking up a biological subject according to one aspect or the present invention includes: a base on which a container having an upper surface opening is mounted, the container accommodating the biological subject and a culture medium; a head placed above the base and movable in a horizontal direction and an up-and-down direction, and having a tip including a suction hole that sucks the biological subject from the container and being attached to a lower end of the head, the head including a suction mechanism that generates suction force in the suction hole; a control unit configured to cause the tip to perform a suction operation of sucking the biological subject by controlling an operation of the head; and an input unit configured to input information to the control unit, wherein the input unit receives at least one of first information regarding a mode of the container, second information regarding the culture medium, and third information regarding the biological subject as suction control information, and when performing the suction operation, based on the suction control information, the control unit controls a movement amount of the tip attached to the head in the up-and-down direction, and/or a suction amount of sucking the biological subject and the culture medium from the suction hole into the tip.

With this pickup device, the movement amount of the tip in the up-and-down direction and/or the suction amount in the suction operation is controlled according to the suction control information received by the input unit. That is, it is possible to change the mode of the suction operation according to the information regarding the mode of the container, the culture medium, and the biological subject. Therefore, it is possible to set an optimum suction sequence according to the suction control information instead of the uniform suction sequence of the biological subject, and as a result, it is possible to prevent the occurrence of a suction failure.

In the device for picking up a biological subject, preferably, in a case where the first information is information indicating the mode of the container including a plurality of wells that each accommodate the biological subject inside the container, and the second information is information indicating that the culture medium with an amount that causes an upper surface of the culture medium to be at a position higher than an upper end opening edge of the wells is held in the container, when performing the suction operation on the biological subject accommodated in each of the wells, the control unit controls the movement amount of the tip to make a position of the suction hole above the biological subject accommodated in each of the wells and below the upper end opening edge of each of the wells.

In the container of the above mode, the biological subjects are planned to be accommodated in respective wells. With the pickup device, the suction operation is performed in a state where the suction hole enters the well that accommodates the biological subject of the suction target. This makes it possible to make a flow accompanying the suction operation unlikely to occur in the peripheral well adjacent to the well. Therefore, when the suction operation is performed on the biological subject of the suction target, it is possible to prevent the biological subject accommodated in the peripheral well from being sucked incidentally. That is, only the biological subject of the suction target can be sucked into the tip.

In the device for picking up a biological subject, preferably, in a case where the first information is information indicating the mode of the container including a plane portion that allows a plurality of the biological subjects to land on a bottom surface of the container, when performing the suction operation on one of the biological subjects that has landed on the bottom surface, the control unit controls the movement amount of the tip to cause the suction hole to be positioned at a position satisfying a relationship:

$$0 < \text{distance } A < 0.5 \times \text{distance } B$$

where a distance between the suction hole and the one biological subject is A, and a distance between the one biological subject and another biological subject closest to the one biological subject is B.

In the container of the above mode, a plurality of biological subjects can exist on the plane portion of the container bottom surface without intervention of partition walls or the like. In this case, due to the suction flow generated during the suction operation on one biological subject, another adjacent biological subject is likely to be simultaneously sucked. However, as in the relational expression, by bringing the suction hole closer to the biological subject of the suction target such that the distance A is 1/2 or less of the distance B, it is possible to increase the probability that only the biological subject is sucked into the tip.

In the device for picking up a biological subject, preferably, in a case where the second information is information regarding viscosity of the culture medium, and the culture medium to be accommodated in the container includes a first culture medium having predetermined first viscosity and a second culture medium having second viscosity higher than the first viscosity, when performing the suction operation on the biological subject in the second culture medium, the control unit controls the movement amount of the tip to make a position of the suction hole closer to the biological subject as compared to a case where the suction operation is performed on the biological subject in the first culture medium.

Generally, when constant suction force is generated in the suction hole, as the viscosity of the culture medium increases, the range in which the flow associated with the suction can occur tends to become narrower. That is, as the viscosity of the culture medium increases, the suction range tends to become narrower. With the pickup device, when performing the suction operation on the biological subject in the second culture medium having higher viscosity, the suction hole is brought closer to the biological subject than in the first culture medium. Therefore, even if the use of culture media having different viscosity is assumed, it is possible to prevent the occurrence of a suction failure.

In the device for picking up a biological subject, preferably, in a case where the third information is information regarding adhesive strength of the biological subject with respect to a bottom surface of the container, and the biological subject to be accommodated in the container includes a first biological subject that lands on the bottom surface with first adhesive strength and a second biological subject that lands with second adhesive strength stronger than the first adhesive strength, when performing the suction operation on the second biological subject, the control unit controls the movement amount of the tip to make a position of the suction hole closer to the biological subject as compared to a case where the suction operation is performed on the first biological subject, and/or the control unit performs control to make the suction amount by the tip larger as compared to a case where the suction operation is performed on the first biological subject.

Depending on the nature of the biological subject, the adhesive strength with respect to the bottom surface of the container may differ. With the pickup device, when the suction operation is performed on the second biological subject having stronger adhesive strength, the suction hole is brought closer to the biological subject or the suction amount is larger than on the first biological subject. That is, the suction force acting on the second biological subject during the suction operation is made larger than on the first biological subject. Therefore, even when the suction of the biological subject having different adhesive strength with respect to the bottom surface is assumed, it is possible to prevent the occurrence of suction failures.

The present invention can provide a device for picking up a biological subject that can prevent a suction failure when causing the tip to suck the biological subject accommodated in the container.

**Claims**

1. A device for picking up a biological subject, the device comprising:

   a base on which a container having an upper surface opening is mounted, the container accommodating the biological subject and a culture medium;
   a head placed above the base and movable in a horizontal direction and an up-and-down direction, and having a tip including a suction hole that sucks the biological subject from the container and being attached to a lower end of the head, the head including a suction mechanism that generates suction force in the suction hole;
a control unit configured to cause the tip to perform a suction operation of sucking the biological subject by controlling an operation of the head; and
an input unit configured to input information to the control unit,
wherein the input unit receives at least one of first information regarding a mode of the container, second information regarding the culture medium, and third information regarding the biological subject as suction control information, and
when performing the suction operation, based on the suction control information, the control unit controls a movement amount of the tip attached to the head in the up-and-down direction, and/or a suction amount of sucking the biological subject and the culture medium from the suction hole into the tip.

2. The device for picking up a biological subject according to claim 1, wherein
in a case where the first information is information indicating the mode of the container including a plurality of wells that each accommodate the biological subject inside the container, and
the second information is information indicating that the culture medium with an amount that causes an upper surface of the culture medium to be at a position higher than an upper end opening edge of the wells is held in the container,
when performing the suction operation on the biological subject accommodated in each of the wells, the control unit controls the movement amount of the tip to make a position of the suction hole above the biological subject accommodated in each of the wells and below the upper end opening edge of each of the wells.

3. The device for picking up a biological subject according to claim 1, wherein
in a case where the first information is information indicating the mode of the container including a plane portion that allows a plurality of the biological subjects to land on a bottom surface of the container,
when performing the suction operation on one of the biological subjects that has landed on the bottom surface,
the control unit controls the movement amount of the tip to cause the suction hole to be positioned at a position satisfying a relationship:

$$0 < \text{distance A} < 0.5 \times \text{distance B}$$

where a distance between the suction hole and the one biological subject is A, and a distance between the one biological subject and another biological subject closest to the one biological subject is B.

4. The device for picking up a biological subject according to claim 1, wherein
in a case where the second information is information regarding viscosity of the culture medium, and the culture medium to be accommodated in the container includes a first culture medium having predetermined first viscosity and a second culture medium having second viscosity higher than the first viscosity,
when performing the suction operation on the biological subject in the second culture medium, the control unit controls the movement amount of the tip to make a position of the suction hole closer to the biological subject as compared to a case where the suction operation is performed on the biological subject in the first culture medium.

5. The device for picking up a biological subject according to claim 1, wherein
in a case where the third information is information regarding adhesive strength of the biological subject with respect to a bottom surface of the container, and the biological subject to be accommodated in the container includes a first biological subject that lands on the bottom surface with first adhesive strength and a second biological subject that lands with second adhesive strength stronger than the first adhesive strength,
when performing the suction operation on the second biological subject,
the control unit controls the movement amount of the tip to make a position of the suction hole closer to the biological subject as compared to a case where the suction operation is performed on the first biological subject, and/or
the control unit performs control to make the suction amount by the tip larger as compared to a case where the suction operation is performed on the first biological subject.

FIG.1

## FIG.2A

631 HEAD RAISING AND LOWERING MOTOR

63

632 PLUNGER RAISING AND LOWERING MOTOR

61R

61

10 { 11 / 12 }

111

121

112

122

11P

113

123

t

## FIG.2B

11 } 10

12

112

122

11P

123

t

113

## FIG.2C

122

11P

123

112

H

t

113

q

FIG.3A

10

11(112)

12(122)

123

11P

a2

a1

113

a3

13

E

t

C

g

a4

FIG.3B

(1)    (2)    (3)    (4)

HEIGHT 0.3

HEIGHT 0.2

HEIGHT 0.1

| 0.2 | 0.1 | 0 | 0.1 | 0.2 |

DISTANCE

(1) 60%-70%   (2) 70%-80%   (3) 80%-90%   (4) 90%-100%

FIG.4

## FIG.5A

## FIG.5B

FIG.6

FIG.7A

10

11

12

11P

E1

C(t)

C(n)

113

A

13

B

A > 0.5 × B

FIG.7B

10

11

12

11P

E2

C(t)

C(n)

113

A

13

t

B

0 < A < 0.5 × B

FIG.8A

FIG.8B

EP 3 739 035 A1

# FIG.9

FIG.10

# FIG.11

74

| SETTING CONDITION | DETAILS |
|---|---|
| TIP | S-type |
| CONTAINER | LATTICE-SHAPED CONTAINER |
| CULTURE MEDIUM | GEL-LIKE CULTURE MEDIUM |
| CELL SIZE | 0.1 |
| SUCTION SEQUENCE | SEQUENCE A |

| SUCTION SEQUENCE | DETAILS |
|---|---|
| SEQUENCE NAME | SEQUENCE A |
| SUCTION HEIGHT | 0.1 |
| SUCTION AMOUNT | 1.0 |

# FIG.12

```
        ( START )
            |
            v
┌──────────────────────────┐
│ RECEIVE INPUT OF SUCTION │ ~S1
│    CONTROL INFORMATION   │
└──────────────────────────┘
            |
            v
┌──────────────────────────┐
│  SELECT SUCTION SEQUENCE │ ~S2
└──────────────────────────┘
            |
            v
┌──────────────────────────┐
│ CAPTURE IMAGE OF CONTAINER│ ~S3
└──────────────────────────┘
            |
            v
┌──────────────────────────┐
│      PERFORM IMAGE       │ ~S4
│ PROCESSING/RECOGNIZE CELL │
└──────────────────────────┘
            |
            v
┌──────────────────────────┐
│   ATTACH NUMBERING n     │ ~S5
│  TO CELL TO BE SUCKED    │
└──────────────────────────┘
            |
            v
┌──────────────────────────┐
│           n=1            │ ~S6
└──────────────────────────┘
            |
            v
┌──────────────────────────┐
│  MOVE HEAD TO POSITION   │ ~S7
│    ABOVE n-TH CELL       │
└──────────────────────────┘
            |
            v
┌──────────────────────────┐
│       LOWER HEAD         │ ~S8
└──────────────────────────┘
            |
            v
┌──────────────────────────┐
│   SUCK CELL WITH TIP     │ ~S9
└──────────────────────────┘
            |                        ┌──────────┐ S12
            v                        │  n=n+1   │
┌──────────────────────────┐         └──────────┘
│       RAISE HEAD         │ ~S10
└──────────────────────────┘
            |
            v
         /‾‾‾‾‾‾\  S11
        < IS SUCTION >──── NO
         \ COMPLETED?/
            |
          YES
            v
         ( END )
```

## INTERNATIONAL SEARCH REPORT

| | International application No. |
|---|---|
| | PCT/JP2018/046339 |

| A. CLASSIFICATION OF SUBJECT MATTER |
|---|
| Int.Cl. C12M1/26(2006.01)i, G01N35/10(2006.01)i |
| According to International Patent Classification (IPC) or to both national classification and IPC |

| B. FIELDS SEARCHED |
|---|
| Minimum documentation searched (classification system followed by classification symbols) |
| Int.Cl. C12M1/26, G01N35/10 |

| Documentation searched other than minimum documentation to the extent that such documents are included in the fields searched |
|---|
| Published examined utility model applications of Japan      1922–1996 |
| Published unexamined utility model applications of Japan    1971–2019 |
| Registered utility model specifications of Japan            1996–2019 |
| Published registered utility model applications of Japan    1994–2019 |

| Electronic data base consulted during the international search (name of data base and, where practicable, search terms used) |
|---|
| JSTPlus/JMEDPlus/JST7580(JDreamIII), WPIDS/WPIX(STN) |

| C. DOCUMENTS CONSIDERED TO BE RELEVANT |
|---|

| Category* | Citation of document, with indication, where appropriate, of the relevant passages | Relevant to claim No. |
|---|---|---|
| Y | WO 2015/087371 A1 (YAMAHA MOTOR CO., LTD.) 18 June 2015, paragraphs [0012]–[0019], [0046]–[0048], [0063]–[0068], [0083]–[0090], claim 1, fig. 1, 4, 6, 9 <br> & US 2016/0304821 A1, fig. 1, 4, 6, 9, paragraphs [0043]–[0049], [0077]–[0079], [0084]–[0089], [0104]–[0111] & EP 3081632 A1 & CN 105814188 A | 1–5 |
| Y | WO 2017/110005 A1 (YAMAHA MOTOR CO., LTD.) 29 June 2017, paragraphs [0001], [0051]–[0069], fig. 10 (Family: none) | 1–5 |

☒ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12.02.2019 | 26.02.2019 |

| Name and mailing address of the ISA/ | Authorized officer |
|---|---|
| Japan Patent Office <br> 3-4-3, Kasumigaseki, Chiyoda-ku, <br> Tokyo 100-8915, Japan | Telephone No. |

Form PCT/ISA/210 (second sheet) (January 2015)

## INTERNATIONAL SEARCH REPORT

| | International application No. |
|---|---|
| | PCT/JP2018/046339 |

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2017-023112 A (YAMAHA MOTOR CO., LTD.) 02 February 2017, paragraphs [0053]-[0062] & US 2018/0203028 A1, paragraphs [0060]-[0069] & WO 2017/017990 A1 & EP 3330366 A1 & CN 107922907 A | 1-5 |
| Y | WO 2017/110004 A1 (YAMAHA MOTOR CO., LTD.) 29 June 2017, paragraphs [0073]-[0081] (Family: none) | 1-5 |
| Y | JP 2003-083851 A (OLYMPUS OPTICAL CO., LTD.) 19 March 2003, paragraph [0019] (Family: none) | 1-5 |
| Y | JP 5-099932 A (ALOKA CO., LTD.) 23 April 1993, paragraph [0005] & US 5452619 A, column 1, lines 49-60 & WO 1993/007494 A1 & EP 606480 A1 | 1-5 |
| Y | WO 2010/022391 A2 (AZTE ARIZONA TECHNOLOGY ENTERPRISES) 25 February 2010, page 33, lines 12-14 (Family: none) | 1-5 |
| Y | JP 7-083939 A (ALOKA CO., LTD.) 31 March 1995, paragraphs [0011], [0018], [0019] (Family: none) | 1-5 |
| Y | JP 2016-106623 A (TOSOH CORPORATION) 20 June 2016, paragraph [0039] (Family: none) | 1-5 |
| A | JP 2014-204671 A (COVALENT MATERIALS CORPORATION) 30 October 2014, fig. 16 (Family: none) | 1-5 |
| A | JP 2006-149226 A (FUJITSU LIMITED) 15 June 2006, fig. 9 & US 2006/0110820 A1, fig. 9 & EP 1666581 A1 | 1-5 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015087371 A